# EUROPEAN PATENT APPLICATION

(11) **EP 4 847 620 A1**
(43) Date of publication of application: **02.12.2026**
(21) Application number: 24919581.9
(22) Date of filing: 08.05.2024
(51) Int. Cl.: C12N 15/54, C12N 15/85, C12N 5/10, A61P 37/04, A61K 48/00

(54) **TRANSPOSASE AND USE THEREOF**

(30) Priority: 23.01.2024 CN 202410095479
(71) Applicant: Triarm Inc., San Mateo, CA 94404 (US); Triarm Therapeutics (Shanghai) Co., Ltd., Shanghai 201203 (CN)
(72) Inventor: LI, Kaiming, Shanghai 201203 (CN); LU, Jinhua, Shanghai 201203 (CN); ZHANG, Jie, Shanghai 201203 (CN); WENG, Jingjie, Shanghai 201203 (CN)
(74) Representative: Elzaburu S.L.P.
(86) International application number: PCT/CN2024/091741
(87) International publication number: WO 2025/156492

(57) **Abstract**

Disclosed is a transposase polypeptide. The transposase polypeptide is derived from a Sleeping Beauty (SB) transposase, and compared to the amino acid sequence at positions 100-180 of the unmutated SB transposase shown in SEQ ID NO: 2, the amino acid sequence of the transposase polypeptide comprises at least one mutated amino acid residue.

## Description

### TECHNICAL FIELD

The present application relates to the field of biomedicine, specifically to a transposase polypeptide and its uses.

### BACKGROUND

Genetic engineering and gene therapy generally involve the transfer of nucleic acids into target cells or genetic modification of target cells. Currently, this process can be carried out using viral vectors, non-viral vectors (such as naked DNA plasmids, RNA, or RNP), and transposon systems. Compared with virus vector-mediated gene delivery, transposon systems offer certain advantages, such as low cost and safety. A transposon system consists of a transposon element and a transposase, which recognizes specific sequences within the transposon and mediates insertion of DNA into the cellular genome. The Sleeping Beauty transposon system has been widely used for various applications in vitro, in vitro and in vivo genetic engineering. Several polypeptides derived from the original SB transposase have been identified and shown to possess varying levels of transposition activity. These transposase variants were obtained through random mutagenesis followed by screening; therefore, the mechanisms underlying their differing transposition activities are not well understood. Generally speaking, the protein stability of the transposase, the efficiency of its transport from the cytoplasm to the nucleus, its ability to recognize the target transposon, cleavage of the target sequence, and integration of the transposon may all influence the final transposition efficiency (Z. Izsvak and Zoltan Ivics. Sleeping Beauty Transposition: Biology and Applications for Molecular Therapy. MOLECULAR THERAPY Vol. 9, No. 2, February 2004). It has been reported in the literature that hyperactive transposases can be obtained by optimizing a specific step in the transposition process-for example, by improving the binding affinity of the transposase for target DNA (Voigt, F. et al. Sleeping beauty transposase structure allows rational design of hyperactive variants for genetic engineering. Nat. Commun. 7:11126. Doi: 10.1038/ncomms11126 (2016*)*). Although transposases obtained through random screening exhibit high transposition efficiency, their unclear mechanisms may affect transposon integration sites and the permissible size of transgenes.

Therefore, there remains a need in the art for methods to develop new transposases with clear mechanisms and high predictability, as well as new transposases exhibiting diverse transposition activities.

### SUMMARY

The present application identifies transposases having different transposition activities based on their activity in the critical step of cleaving a transposon, wherein the mechanism is clear, and the resulting enzymes can also be used to screen corresponding transposon sequences, thereby forming an efficient and safe transposon system. Obtaining the desired transposase polypeptide by improving a specific step offers greater predictability and facilitates the safety assessment of products derived therefrom.

The present application employs an alanine scanning (ala-screening) method to generate mutants of the SB transposase, particularly in a region that has not been actively studied previously. These mutants were evaluated to exhibit varying excision activities, and the screening method is rapid and simple. Furthermore, these mutants containing single or combined mutations (Tri-FITase, fine-tuned transposase for *in vitro*, *in vivo* and *in vivo* applications) were found to possess different transposition activities and thus can be used in various contexts-for example, in T cell genetic engineering, which may require transposases with either higher or lower activity, the capacity to carry genetic material of varying sizes, and/or distinct integration profiles.

In one aspect, the present application provides a transposase polypeptide whose amino acid sequence comprises at least one mutated amino acid residue, compared to the amino acid sequence from positions 100 to 180 of the unmutated SB transposase as set forth in SEQ ID NO: 2.

In some embodiments, the amino acid sequence of the transposase polypeptide comprises 1 to 20 mutated amino acid residues.

In some embodiments, the amino acid sequence of the transposase polypeptide comprises at least two mutated amino acid residues.

In some embodiments, the mutation site(s) of the at least one mutated amino acid residue is located within positions 110 to 120 and/or positions 170 to 180 of the amino acid sequence as set forth in SEQ ID NO: 2.

In some embodiments, the mutated amino acid residue(s) is located at position 114, 116, 177, and/or 178 of the amino acid sequence as set forth in SEQ ID NO: 2.

In some embodiments, compared to the amino acid sequence of the unmutated SB transposase as set forth in SEQ ID NO: 2, the amino acid sequence of the transposase polypeptide comprises a mutation at amino acid position G114.

In some embodiments, compared to the amino acid sequence of the unmutated SB transposase set forth in SEQ ID NO: 2, the amino acid sequence of the transposase polypeptide comprises a mutation at amino acid position S116.

In some embodiments, compared to the amino acid sequence of the unmutated SB transposase as set forth in SEQ ID NO: 2, the amino acid sequence of the transposase polypeptide comprises a mutation at amino acid position K177.

In some embodiments, compared to the amino acid sequence of the unmutated SB transposase as set forth in SEQ ID NO: 2, the amino acid sequence of the transposase polypeptide comprises a mutation at amino acid position P178.

In some embodiments, the at least one mutated amino acid residue is mutated to an alanine residue. In some embodiments, all mutated amino acid residues are alanine residues.

In some embodiments, compared to the unmutated SB transposase as set forth in SEQ ID NO: 2, the amino acid sequence of the transposase polypeptide comprises an amino acid mutation selected from the group consisting of: G114A, S116A, K177A, and P178A.

In some embodiments, compared to the amino acid sequence of the unmutated SB transposase as set forth in SEQ ID NO: 2, the amino acid sequence of the transposase polypeptide comprises a mutation at amino acid position G114 to alanine (G114A).

In some embodiments, compared to the amino acid sequence of the unmutated SB transposase as set forth in SEQ ID NO: 2, the amino acid sequence of the transposase polypeptide comprises a mutation at amino acid position S116 to alanine (S116A).

In some embodiments, compared to the amino acid sequence of the unmutated SB transposase as set forth in SEQ ID NO: 2, the amino acid sequence of the transposase polypeptide comprises a mutation at amino acid position K177 to alanine (K177A).

In some embodiments, compared to the amino acid sequence of the unmutated SB transposase as set forth in SEQ ID NO: 2, the amino acid sequence of the transposase polypeptide comprises a mutation at amino acid position P178 to alanine (P178A).

In certain embodiments, compared to the unmutated SB transposase as set forth in SEQ ID NO: 2, the amino acid sequence of the transposase polypeptide comprises a combination of amino acid mutations selected from the group consisting of:
i) G114A, S116A;
ii) G114A, K177A;
iii) G114A, P178A;
iv) S116A, K177A;
v) S116A, P178A; and
vi) K177A, P178A.

In another aspect, the present application provides a transposase polypeptide comprising a sequence having at least 90% identity to SEQ ID NO: 2, wherein the transposase polypeptide comprises an alanine at the position corresponding to position 114 and/or an alanine at the position corresponding to position 116.

In some embodiments, the transposase polypeptide comprises the amino acid sequence of any one of SEQ ID NO: 1 or SEQ ID NOs: 3-11.

In another aspect, the present application provides a polynucleotide molecule comprising a nucleic acid sequence encoding the transposase polypeptide described herein.

In some embodiments, it comprises RNA or DNA.

In some embodiments, the DNA comprises a promoter for expression of the transposase polypeptide.

In some embodiments, the DNA comprises a promoter for expression of the transposase polypeptide in mammalian cells.

In some embodiments, the molecule is mRNA.

In some embodiments, the polynucleotide molecule comprises a 5' cap, an IRES motif, and/or a poly(A) sequence.

In some embodiments, the polynucleotide molecule comprises a poly(A) sequence of 20 to 300 nucleotides.

In another aspect, the present application provides a construct comprising the polynucleotide molecule described herein and a regulatory sequence, wherein the regulatory sequence is operably linked to the expressible polynucleotide to allow expression thereof.

In another aspect, the present application provides a cell comprising the transposase polypeptide described herein, the polynucleotide molecule described herein, and/or the construct described herein.

In some embodiments, the cell comprises a mammalian cell.

In some embodiments, the cell comprises a human cell.

In some embodiments, the cell is autologous.

In some embodiments, the cell is allogeneic.

In some embodiments, the cell comprises a stem cell or an induced pluripotent stem (iPS) cell.

In some embodiments, the cell comprises an immune effector cell.

In some embodiments, the cell comprises a natural killer (NK) cell, a T cell, or a precursor of an NK cell or a T cell.

In some embodiments, the cell comprises the transposase polypeptide described herein.

In some embodiments, the cell comprises mRNA or DNA encoding the transposase polypeptide described herein.

In another aspect, the present application provides a transposon system comprising:
(a) a transposon unit comprising an inverted terminal repeat (ITR) or a direct terminal repeat (DTR), wherein the inverted terminal repeat (ITR) or direct terminal repeat (DTR) flanks a target sequence to be inserted into the genome of a target cell; and
(b) the transposase polypeptide described herein, the polynucleotide molecule described herein, and/or the construct described herein.

In another aspect, the present application provides an use of the transposon system described herein for gene delivery to target cells in vitro.

In another aspect, the present application provides a method of genetically engineering a cell, comprising the steps of:
(a) transfecting a target cell with the transposon system described herein;
(b) culturing the target cell under conditions permitting culture of the target cell to produce an engineered cell.

In some embodiments, the method further comprises: (c) isolating the engineered cells.

In some embodiments, the target sequence encodes a screenable or selectable marker.

In some embodiments, the target sequence encodes a therapeutic polypeptide or an inhibitory nucleic acid.

In some embodiments, the target sequence encodes an antibody, a T cell receptor (TCR), or a chimeric antigen receptor (CAR).

In some embodiments, the target sequence encodes a CAR.

In some embodiments, the CAR targets a tumor antigen.

In some embodiments, the tumor antigen is CD19, CD20, ROR1, CD22 carcinoembryonic antigen, alpha-fetoprotein, CA-125, 5T4, MUC-1, epithelial tumor antigen, prostate-specific antigen, melanoma-associated antigen, mutated p53, mutated ras, HER2/Neu, folate-binding protein, HIV-1 envelope glycoprotein gp120, HIV-1 envelope glycoprotein gp41, GD2, CD123, CD33, CD138, CD23, CD30, CD56, c-Met, mesothelin, GD3, HERV-K, IL-11Rα, κ chain, λ chain, CSPG4, ERBB2, EGFRvIII, VEGFR2, a combination of HER2-HER3, or a combination of HER1-HER2. In some embodiments, the target cell comprises a mammalian cell.

In some embodiments, the target cell comprises a human cell.

In some embodiments, the target cell is autologous.

In some embodiments, the target cell is allogeneic.

In some embodiments, the target cell comprises a stem cell or an induced pluripotent stem (iPS) cell.

In some embodiments, the target cell comprises an immune effector cell or a precursor thereof. In some embodiments, the target cell comprises a natural killer (NK) cell, a T cell, or a precursor of an NK cell or a T cell.

In some embodiments, transfecting the target cell comprises using a polymer-based, polypeptide-based or lipid-based transfection reagent.

In some embodiments, transfecting the target cell comprises electroporation of the cell.

In some embodiments, the method further comprises:
(a) transfecting a population of cells with the transposon system described herein;
(b) incubating the population under conditions suitable for transposase activity, thereby integrating the target sequence into the genome of the cells and producing a population of engineered cells.

In some embodiments, the method further comprises:
(a) transfecting a population of T cells or T cell precursors with the transposon system described herein, wherein the target sequence encodes a CAR or a TCR;
(b) incubating the population under conditions suitable for transposase activity, thereby integrating the CAR into the genome of the cells and producing a population of engineered T cells or T cell precursors.

In some embodiments, the method further comprises: (c) culturing the engineered cells in a medium that selectively enhances proliferation of CAR- or TCR-expressing T cells.

In another aspect, the present application provides a pharmaceutical composition comprising the transposase polypeptide described herein, the polynucleotide molecule described herein, and/or the construct described herein, together with a pharmaceutically acceptable carrier and/or excipient. In another aspect, the present application provides a kit comprising
(a) a transposon unit comprising an inverted terminal repeat (ITR) or DTR, wherein said inverted terminal repeat (ITR) or DTR flanks a target sequence to be inserted into the genome of a target cell; and
(b) the transposase polypeptide described herein, the polynucleotide molecule described herein, and/or the construct described herein.

In another aspect, the present application provides a use of the transposase polypeptide described herein, the polynucleotide molecule described herein, the construct described herein, the cell described herein, the transposon system described herein, and/or the pharmaceutical composition described herein in the manufacture of a medicament for treating a disease or disorder.

In some embodiments, the disease or disorder is cancer.

In some embodiments, the disease or disorder is an infectious disease.

In another aspect, the present application provides a compound for treating a disease or disorder, wherein the compound comprises the transposase polypeptide described herein, the polynucleotide molecule described herein, the construct described herein, the cell described herein, the transposon system described herein, the pharmaceutical composition described herein, and/or the kit described herein.

Other aspects and advantages of the present application will be readily apparent to those skilled in the art from the detailed description below. Only exemplary embodiments of the present application are shown and described in the detailed description below. As will be recognized by those skilled in the art, the content of the present application enables those skilled in the art to make modifications to the specific embodiments disclosed herein without departing from the spirit and scope of the invention described herein. Accordingly, the description in the drawings and specification of the present application is merely exemplary and not limiting.

### BRIEF DESCRIPTION OF THE DRAWINGS

The specific features of the invention to which the present application relates are set forth in the appended claims. The features and advantages of the invention to which the present application relates may be more fully understood with reference to the exemplary embodiments and drawings described in detail below. The drawings are briefly described below:
Figure 1A shows a schematic diagram of an excision assay distinguishing SB transposases with known transposition activity according to the present application.
Figure 1B shows the results of excision assays performed with three commonly used SB transposases.

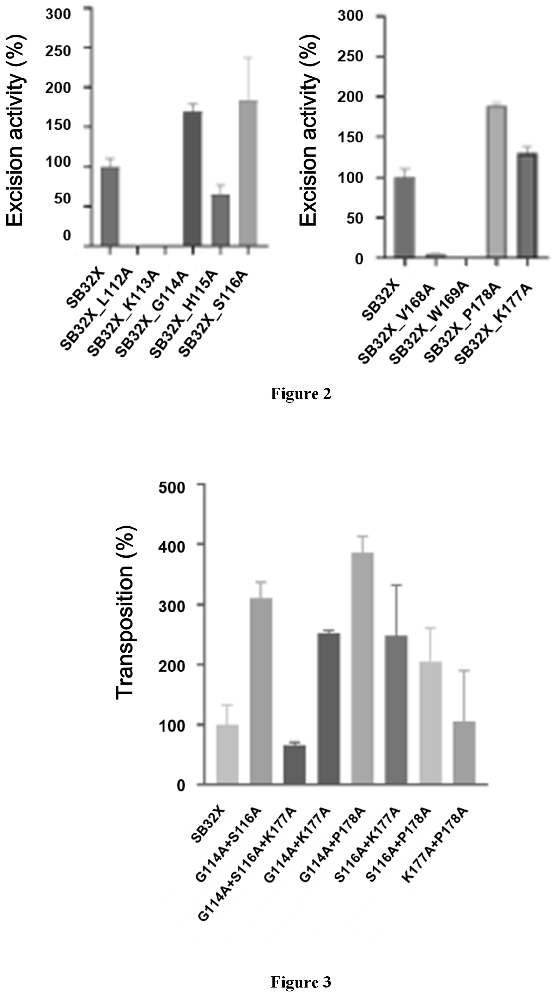

Figure 2 shows that transposase mutants generated by alanine scanning exhibit different transposase cleavage activities.
Figure 3 shows the results of a transposase transposition assay performed with the Tri-FITase of the present application in HeLa cells.

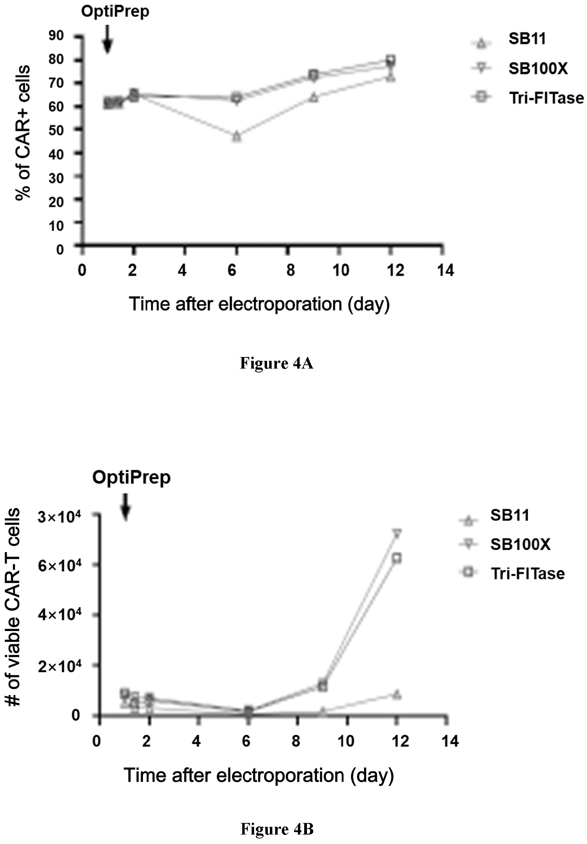

Figures 4A-4B show that the Tri-FITase of the present application achieves expression of chimeric antigen receptor (CAR) in T cells.

### DETAILED DESCRIPTION

The implementation of the invention of the present application is described below with reference to specific embodiments, and those skilled in the art can easily understand other advantages and effects of the invention of the present application from the disclosure of this specification.

### Definitions of Terms

In the present application, the term "transposase" generally refers to an enzyme that is a component of a functional nucleic acid-protein complex capable of transposition and mediates transposition. The term "transposase" may also include integrases derived from retrotransposons or retroviruses. As used herein, "transposition reaction" generally refers to the reaction of transposon insertion into target nucleic acid. The main components in a transposition reaction are the transposon and the transposase or integrase. For example, the transposase system according to the present invention is preferably derived from the so-called "Sleeping Beauty (SB)" transposase. In certain aspects, the transposase is an engineered enzyme with improved properties, such as enhanced enzymatic function. Specific examples of engineered SB transposases include, but are not limited to, SB10, SB11, or SB100×SB transposase (see, e.g., Mates et al., Nat. Gen. 2009, incorporated herein by reference). Other transposon systems may be used, such as Ty1 (Devine and Boeke, 1994 and WO 95/23875), Tn7 (Craig, 1996), Tn10 and IS10 (Kleckner et al., 1996), Mariner transposase (Lampe et al., 1996), Tc1 (Vos et al., 1996), Tn5 (Park et al., 1992), P element (Kaufman and Rio, 1992) and Tn3 (Ichikawa and Ohtsubo, 1990), bacterial insertion sequences (Ohtsubo and Sekine, 1996), retroviruses (Varmus and Brown, 1989), and yeast retrotransposons (Boeke, 1989).

In the present application, the term "transposon unit" refers to a nucleic acid construct comprising a transposon gene sequence and a target sequence to be introduced into the genome of a target cell. Typically, the transposon unit is nucleic acid and may be any form of vector suitable for transposition.

In the present application, the term "inverted terminal repeat" refers to a sequence located at one end of a transposon unit that can be cleaved by a transposase polypeptide when used in combination with a complementary sequence located at the opposite end of the vector or transposon unit. The pair of inverted terminal repeats is involved in the transposition activity of the transposon of the transposon unit disclosed herein, particularly concerning DNA addition to or removal from target DNA, as well as excision and integration. In one example, at least one pair of inverted terminal repeats appears to be the minimal sequence required for transposition activity in a plasmid. In another example, the transposon unit disclosed herein may comprise at least two, three, or four pairs of inverted terminal repeats. As understood by those skilled in the art, to facilitate easy cloning, the essential terminal sequences should be as short as possible, thereby containing as few inverted repeats as possible. Thus, in one example, the transposon unit disclosed herein may contain no more than one, two, three, or four pairs of inverted terminal repeats. In one example, the transposon unit disclosed herein may include only one inverted terminal repeat. Although not wishing to be bound by theory, it is contemplated that having more than one inverted terminal repeat may be disadvantageous, as it could lead to non-specific binding of the transposase to multiple inverted terminal repeats, resulting in removal of desired sequences or insertion of undesired sequences. The inverted terminal repeats disclosed herein may form perfect inverted terminal repeats (or interchangeably referred to as "perfect inverted repeats") or imperfect inverted terminal repeats (or interchangeably referred to as "imperfect inverted repeats"). As used herein, the term "perfect inverted repeat" refers to two identical DNA sequences placed in opposite orientations. The above description of transposon units with ITRs also applies to transposon units with DTRs.

Transposon systems that can be used together with the transposase polypeptides/nucleic acids of the present invention are disclosed, for example, in WO2017/050448A1, which is incorporated herein by reference.

In the present application, the terms "chimeric antigen receptor" or "CAR" generally refer to a group of polypeptides, typically two in the simplest embodiment, which, when present in an immune effector cell, confer specificity of the cell toward a target cell (typically a cancer cell) and generate an intracellular signal. In some embodiments, the CAR comprises at least one extracellular antigen-binding domain (e.g., VHH, scFv, or a portion thereof), a transmembrane domain, and a cytoplasmic signaling domain (also referred to herein as an "intracellular signaling domain") comprising a functional signaling domain derived from a stimulatory molecule and/or a costimulatory molecule as defined below. In some embodiments, the group of polypeptides resides within the same polypeptide chain (e.g., comprising a chimeric fusion protein). In some embodiments, the group of polypeptides is discontinuous, e.g., located on different polypeptide chains. In some aspects, the group of polypeptides includes a dimerization switch that, in the presence of a dimerizing molecule, couples the polypeptides together, e.g., coupling the antigen-binding domain to the intracellular signaling domain. In one aspect, the stimulatory molecule of the CAR is the ζ chain associated with the T cell receptor complex. In one aspect, the cytoplasmic signaling domain comprises a primary signaling domain (e.g., the primary signaling domain of CD3-ζ). In one aspect, the cytoplasmic signaling domain further comprises one or more functional signaling domains derived from at least one costimulatory molecule as defined below. In one aspect, the costimulatory molecule may be selected from 4-1BB (i.e., CD137), CD27, ICOS, and/or CD28. In one aspect, the CAR comprises a chimeric fusion protein that may include an extracellular antigen recognition domain, a transmembrane domain, and an intracellular signaling domain comprising a functional signaling domain derived from a stimulatory molecule. In one aspect, the CAR comprises a chimeric fusion protein that may include an extracellular antigen recognition domain, a transmembrane domain, and an intracellular signaling domain comprising a functional signaling domain derived from a costimulatory molecule and a functional signaling domain derived from a stimulatory molecule. In one aspect, the CAR comprises a chimeric fusion protein that may include an extracellular antigen recognition domain, a transmembrane domain, and an intracellular signaling domain comprising functional signaling domains derived from one or more costimulatory molecules and a functional signaling domain derived from a stimulatory molecule. In one aspect, the CAR comprises a chimeric fusion protein that may include an extracellular antigen recognition domain, a transmembrane domain, and an intracellular signaling domain comprising at least two functional signaling domains derived from one or more costimulatory molecules and a functional signaling domain derived from a stimulatory molecule. In one aspect, the CAR comprises an optional leader sequence at the amino terminus (N-ter) of the CAR fusion protein. In one aspect, the CAR further comprises a leader sequence at the N-terminus of the extracellular antigen recognition domain, wherein the leader sequence is optionally cleaved from the antigen recognition domain (e.g., VHH) during cellular processing and directs the CAR to the cell membrane.

In the present application, the term "antibody" generally is used in its broadest sense and specifically encompasses monoclonal antibodies, polyclonal antibodies, dimers, multimers, multispecific antibodies (e.g., bispecific antibodies), and antibody fragments, as long as they exhibit the desired biological activity (Miller et al. (2003) Jour. of Immunology 170:4854-4861). Antibodies may be murine, human, humanized, chimeric, or derived from other species.

In the present application, the term "homology" is generally equivalent to sequence "identity" Homologous sequences may include amino acid sequences that are at least 80%, 85%, 90%, 99.1%, 99.2%, 99.3%, 99.4%, 99.5%, 99.6%, 99.7%, 99.8% or 99.9% identical to the subject sequence. Typically, homologs will contain the same active sites, etc., as the subject amino acid sequence. Homology may be considered in terms of similarity (i.e., amino acid residues with similar chemical properties/functions) or expressed in terms of sequence identity. In the present application, a sequence having a percentage identity to any of the SEQ ID NOs of amino acid or nucleotide sequences mentioned refers to a sequence having the stated percentage identity over the entire length of the referenced SEQ ID NO.

To determine sequence identity, sequence alignment may be performed using various methods known to those skilled in the art, for example, using BLAST, BLAST-2, ALIGN, NEEDLE, or Megalign (DNASTAR) software. Those skilled in the art can determine appropriate parameters for alignment, including any algorithm necessary to achieve optimal alignment across the full length of the sequences being compared.

In the present application, the terms "upstream" and "downstream" are functional definitions that generally refer to the direction or polarity along a coding nucleotide sequence strand. The term "upstream" means that a nucleotide is located in the 5' direction of a given polynucleotide sequence, i.e., toward the initiating nucleotide. With respect to amino acid sequences, the term "upstream" refers to amino acids located in the N-terminal direction, i.e., toward the beginning of the polypeptide chain.

In the present application, the term "isolated nucleic acid molecule" generally refers to nucleotides, deoxyribonucleotides, ribonucleotides, or analogs thereof, in isolated form, of any length, that have been separated from their natural environment or synthetically produced.

In the present application, the term "construct" generally refers to a nucleic acid molecule capable of self-replication in a suitable host, which transfers the inserted nucleic acid molecule into and/or between host cells. The construct may comprise vectors primarily used for inserting DNA or RNA into cells, vectors primarily used for replicating DNA or RNA, and vectors primarily used for transcription and/or translation of DNA or RNA. The vectors also include those possessing multiple functions described above. The construct may be a polynucleotide that, when introduced into a suitable host cell, is capable of being transcribed and translated into a polypeptide. Typically, the desired expression product can be produced by culturing suitable host cells containing the vector.

In the present application, the terms "host cell" or "cell" generally refer to individual cells, cell lines, or cell cultures that can or do contain a vector comprising the isolated nucleic acid molecule described herein, or that are capable of expressing the isolated antigen-binding fragment described herein. The host cell may include progeny of a single host cell. Due to natural, accidental, or intentional mutations, progeny cells may not necessarily be morphologically or genomically identical to the original parental cell, but are acceptable as long as they can express the isolated antigen-binding fragment described herein. The host cell can be obtained by transfecting cells in vitro using the vector described herein. The host cell may be a prokaryotic cell (e.g., Escherichia coli) or a eukaryotic cell (e.g., yeast cells, such as COS cells, Chinese hamster ovary (CHO) cells, HeLa cells, HEK293 cells, COS-1 cells, NS0 cells, or myeloma cells). For example, the host cell may be an Escherichia coli cell. For example, the host cell may be a yeast cell. For example, the host cell may be a mammalian cell. For example, the mammalian cell may be a CHO-K1 cell.

In the present application, the terms "T cell" or "T lymphocyte" may refer to any T cell, such as cultured T cells, e.g., primary T cells, or T cells from cultured T cell lines, e.g., Jurkat, SupTI, etc., or T cells obtained from mammals (preferably primates, species, including monkeys, dogs, or humans). If obtained from mammals, T cells may be derived from numerous sources, including but not limited to blood, bone marrow, lymph nodes, thymus, or other tissues or fluids. T cells may also be enriched or differentiated. T cells may be obtained by maturing hematopoietic stem cells into T cells in vitro or in vivo. In exemplary aspects, T cells are human T cells. In exemplary aspects, T cells are T cells isolated from humans. T cells may be any type of T cell, including NKT cells, and may be at any developmental stage, including but not limited to CD4⁺/CD8⁺ double-positive T cells; CDA⁺ helper T cells, e.g., Th1 and Th2 cells; CD8⁺ T cells (e.g., cytotoxic T cells); peripheral blood mononuclear cells (PBMCs); peripheral blood leukocytes (PBLs); tumor-infiltrating lymphocytes (TILs); memory T cells; naïve T cells; etc. Preferably, T cells are CD8⁺ T cells or CD4⁺ T cells. In some alternative embodiments, the T cells are allogeneic (from a different donor of the same species) relative to the recipient subject who receives or is to receive the cells (e.g., the cells are in the form of a therapeutic composition); in some alternative embodiments, the T cells are autologous (the donor and recipient are the same); in some alternative embodiments, the T cells are syngeneic (the donor and recipient are different but monozygotic twins).

In the present application, the term "immune effector cell" generally refers to immune cells that participate in immune responses and perform effector functions. For example, performing effector functions may include clearing foreign antigens or promoting immune effector responses. Immune effector cells may include plasma cells, T cells, B cells, natural killer (NK) cells, natural killer T (NKT) cells, mast cells, and myeloid-derived phagocytes.

The immune effector cells of the present application may be autologous/autogeneic ("self") or non-autologous ("non-self," e.g., allogeneic, syngeneic, or xenogeneic). In the present application, the term "autologous" generally refers to cells derived from the same subject. "Allogeneic" generally refers to cells from the same species as the comparator but genetically distinct. "Syngeneic" generally refers to cells from a different subject that are genetically identical to the comparator cells. "Xenogeneic" generally refers to cells that are from a species different from that of the cells being compared. In some embodiments, the cells of the present application are autologous or allogeneic.

In the present application, the term "modification" generally refers to an alteration of the state or structure of a cell and/or the alteration in the state or structure of the cell. Such alteration is typically compared to the state or structure of a corresponding unmodified cell, and may include changes in the expression level or function of endogenous genes, for example, downregulation, upregulation, or silencing of endogenous gene expression achieved by genetic engineering techniques such as homologous recombination or CRISPR/Cas9-mediated gene editing, etc; such alteration may also include changes in protein expression, structure, or function in the cell, for example, changes in protein expression, structure, or function resulting from said changes in endogenous gene expression level or function, or changes in protein expression, structure, or function achieved by modulating protein translation or post-translational modifications; such alteration may further include introduction of exogenous genes or expression of exogenous proteins.

In the present application, the terms "nucleic acid," "polynucleotide," or "nucleic acid molecule" generally refer to polymers of deoxyribonucleic acid (DNA) or ribonucleic acid (RNA) in either single-stranded or double-stranded form. Unless specifically limited, the term may include nucleic acids containing analogs of natural nucleotides, which exhibit binding properties similar to those of a reference nucleic acid (e.g., one for which sequence information is provided) and are metabolized in a manner similar to naturally occurring nucleotides. Unless otherwise indicated, a nucleic acid sequence may include conservatively modified variants thereof, such as degenerate codon substitutions, alleles, orthologs, SNPs, complementary sequences, as well as the explicitly stated sequence.

In the present application, the term "operably linked" generally refers to positioning regulatory sequences necessary for expression of a coding sequence in an appropriate location relative to the coding sequence so as to enable expression of the coding sequence. The term "operably linked" may also refer to the arrangement of a coding sequence and regulatory sequences (e.g., promoters, enhancers, and termination elements) within an expression vector. This definition may also apply to the arrangement of nucleic acid sequences of first and second nucleic acid molecules used to generate a hybrid nucleic acid molecule.

In the present application, the term "regulatory sequence" generally includes promoters, enhancers, and other expression regulatory elements (e.g., polyadenylation signals) that regulate transcription or translation of antibody chain genes. Such regulatory sequences are described, for example, in Goeddel, Gene Expression Technology. Methods in Enzymology 185, Academic Press, San Diego, CA (1990). Those skilled in the art will understand that the design of expression vectors, including the selection of regulatory sequences, depends on factors such as the choice of host cell to be transformed and the desired level of protein expression. Regulatory sequences for expression in mammalian host cells may include viral elements that direct high-level protein expression in mammalian cells, such as promoters and/or enhancers derived from cytomegalovirus (CMV), Simian Virus 40 (SV40), adenovirus (e.g., adenovirus major late promoter (AdMLP)), and polyomavirus. Alternatively, non-viral regulatory sequences may be used, such as ubiquitin promoter or β-globin promoter. Furthermore, regulatory elements may contain sequences from different sources, such as the SRα promoter system, which comprises sequences from the SV40 early promoter and the long terminal repeat (LTR) of human T-cell leukemia virus type 1 (Takebe, Y. et al. (1988) Mol. Cell. Biol. 8: 466-472).

In the present application, the term "expression" generally refers to transcription and/or translation of a specific nucleotide sequence.

In the present application, the terms "tumor" and "cancer" are used interchangeably and generally refer to diseases characterized by rapid and uncontrolled growth of abnormal cells. Cancer cells can spread locally or to other parts of the body via the bloodstream and lymphatic system. Examples of various cancers described herein include, but are not limited to, breast cancer, prostate cancer, ovarian cancer, cervical cancer, skin cancer, pancreatic cancer, colorectal cancer, renal cancer, liver cancer, brain cancer, lymphoma, leukemia, and lung cancer. The terms "cancer" or "tumor" include both premalignant and malignant cancers and tumors, and encompass both solid tumors and non-solid tumors.

As used herein, "tumor antigen" includes its meaning known in the art, referring to any molecule expressed on tumor cells (or associated with tumor cell development) that is known or believed to contribute to the tumorigenic properties of the tumor cells. Many tumor antigens are known in the art. Whether a molecule is a tumor antigen can also be determined using techniques and assays well known to those skilled in the art, such as clonogenic assay, transformation assay, in vitro or in vivo tumorigenicity assay, gel migration assay, gene knockout analysis, etc. Preferably, the term "tumor antigen" as used herein refers to a human transmembrane protein, i.e., a cell membrane protein anchored in the lipid bilayer of the cell. The human transmembrane proteins used herein typically contain an "extracellular domain" capable of binding ligands, a lipophilic transmembrane domain, a conserved intracellular domain such as a tyrosine kinase domain, and a carboxy-terminal signaling domain containing several tyrosine residues that can be phosphorylated. Tumor antigens include molecules such as EGFR, HER2/neu, HER3, HER4, EpCAM, CEA, TRAIL, TRAIL receptor 1, TRAIL receptor 2, lymphotoxin β receptor, CCR4, CD19, CD20, CD22, CD28, CD33, CD40, CD80, CSF-1R, CTLA-4, fibroblast activation protein (FAP), hepsin, melanoma-associated chondroitin sulfate proteoglycan (MCSP), prostate-specific membrane antigen (PSMA), VEGF receptor 1, VEGF receptor 2, IGF1-R, TSLP-R, TIE-1, TIE-2, TNF-α, TNF-like weak inducer of apoptosis (TWEAK), or IL-1R.

In the present application, the term "pharmaceutically acceptable" generally refers to compounds, materials, compositions, and/or dosage forms that, within the scope of sound medical judgment, are suitable for contact with human and animal tissues, have a favorable benefit/risk ratio, and do not exhibit excessive toxicity, irritation, allergic response, or other problems or complications. In the present application, the term "pharmaceutically acceptable carrier" generally refers to any and all solvents, dispersion media, preservatives, antioxidants, coatings, isotonic and absorption delaying agents, surfactants, fillers, disintegrants, binders, diluents, lubricants, glidants, pH adjusters, buffers, enhancers, wetting agents, solubilizers, surfactants, antioxidants, etc. that are compatible with pharmaceutical administration. The use of such media and agents for pharmaceutically active substances is well known in the art. The composition may contain additional active compounds that provide supplementary, additive, or enhanced therapeutic functions.

In the present application, the terms "effective amount" or "effective dose" generally refer to an amount sufficient to achieve or at least partially achieve the desired effect. A "therapeutically effective amount" or "therapeutically effective dose" of a drug or therapeutic agent generally refers to any amount of the drug that, when used alone or in combination with another therapeutic agent, promotes disease regression (as evidenced by a reduction in the severity of disease symptoms, an increase in the frequency and duration of symptom-free periods, or prevention of damage or disability caused by the disease).

In the present application, the term "comprising" generally has the meaning of including, encompassing, containing, or incorporating. In certain contexts, it may also mean "being" or "consisting of."

In the present application, the term "about" generally refers to a variation within ±0.5% to 10% of the specified value, such as a variation of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

In the present application, the term "subject" generally refers to a human or non-human animal, including but not limited to cats, dogs, horses, pigs, cows, sheep, rabbits, mice, rats, or monkeys. In the present application, the term "selectable marker gene" refers to a nucleic acid sequence that expresses an activity enabling positive or negative selection for the presence or absence of the sequence in a host cell. A selectable marker sequence or gene does not necessarily exhibit both positive and negative selectability. Important positive selection markers include, for example, antibiotic resistance genes. Selectable markers can be positive, negative, or bifunctional. Positive selectable markers allow selection of cells carrying the marker, whereas negative selectable markers allow selective elimination of cells carrying the marker. Typically, a selectable marker confers resistance to a drug or compensates for a metabolic or catabolic deficiency in the cell. Selectable markers useful in eukaryotic cells include, for example, aminoglycoside phosphotransferases (APH), such as hygromycin phosphotransferase (hyg), neomycin and G418 APH, dihydrofolate reductase (DHFR), thymidine kinase (tk), glutamine synthetase (GS), asparagine synthetase, tryptophan synthase (selection agent indole), histidinol dehydrogenase (selection agent histidinol D), and genes conferring resistance to puromycin, bleomycin, phleomycin, chloramphenicol, Zeocin, and mycophenolic acid. Other selectable markers are described, for example, in WO 92/08796 and WO 94/28143.

### Detailed Description of the Invention

In one aspect, the present application provides a transposase polypeptide derived from Sleeping Beauty (SB) transposase, wherein, compared to the amino acid sequence from positions 100 to 180 of the unmutated SB transposase as set forth in SEQ ID NO: 2, the amino acid sequence of the transposase polypeptide comprises at least one mutated amino acid residue.

In some embodiments, the number of mutated amino acid residues is 1 to 20. For example, the number of mutated amino acid residues is 1, 2, 3, 4, 5, 6, 7, 8, 9, 10, 11, 12, 13, 14, 15, 16, 17, 18, 19, or 20.

In some embodiments, said at least one mutated amino acid residue comprises at least two mutated amino acid residues. For example, the number of mutated amino acid residues is 2 to 20, 2 to 19, 2 to 18, 2 to 17, 2 to 16, 2 to 15, 2 to 14, 2 to 13, 2 to 12, 2 to 11, 2 to 10, 2 to 9, 2 to 8, 2 to 7, 2 to 6, 2 to 5, 2 to 4, or 2 to 3. For example, the number of mutated amino acid residues is 2.

In some embodiments, the mutation site(s) of the at least one mutated amino acid residue is located within amino acid positions 110 to 120 and 170 to 180 of the amino acid sequence as set forth in SEQ ID NO: 2. For example, the mutation site(s) is located at amino acid position(s) 110, 111, 112, 113, 114, 115, 116, 117, 118, 119, 120, 170, 171, 172, 173, 174, 175, 176, 177, 178, 179, and/or 180 of the amino acid sequence as set forth in SEQ ID NO: 2.

In some embodiments, the mutated amino acid residue(s) is located at amino acid positions 114, 116, 177, and/or 178 of the amino acid sequence as set forth in SEQ ID NO: 2.

For example, compared to the unmutated SB transposase amino acid sequence as set forth in SEQ ID NO: 2, the amino acid sequence of the transposase polypeptide comprises a mutation at amino acid position G114. For example, compared to the unmutated SB transposase amino acid sequence as set forth in SEQ ID NO: 2, the amino acid sequence of the transposase polypeptide comprises a mutation at amino acid position S116. For example, compared to the unmutated SB transposase amino acid sequence as set forth in SEQ ID NO: 2, the amino acid sequence of the transposase polypeptide comprises a mutation at amino acid position K177. For example, compared to the unmutated SB transposase amino acid sequence as set forth in SEQ ID NO: 2, the amino acid sequence of the transposase polypeptide comprises a mutation at amino acid position P178.

For example, compared to the unmutated SB transposase amino acid sequence as set forth in SEQ ID NO: 2, the amino acid sequence of the transposase polypeptide comprises mutations at amino acid positions G114 and S116. For example, compared to the unmutated SB transposase amino acid sequence as set forth in SEQ ID NO: 2, the amino acid sequence of the transposase polypeptide comprises mutations at amino acid positions G114 and K177. For example, compared to the unmutated SB transposase amino acid sequence as set forth in SEQ ID NO: 2, the amino acid sequence of the transposase polypeptide comprises mutations at amino acid positions G114 and P178. For example, compared to the unmutated SB transposase amino acid sequence as set forth in SEQ ID NO: 2, the amino acid sequence of the transposase polypeptide comprises mutations at amino acid positions S116 and K177. For example, compared to the unmutated SB transposase amino acid sequence as set forth in SEQ ID NO: 2, the amino acid sequence of the transposase polypeptide comprises mutations at amino acid positions S116 and P178. For example, compared to the unmutated SB transposase amino acid sequence as set forth in SEQ ID NO: 2, the amino acid sequence of the transposase polypeptide comprises mutations at amino acid positions K177 and P178.

In some embodiments, the at least one mutated amino acid residue is mutated to an alanine residue. In some embodiments, all mutated amino acid residues are mutated to alanine residues.

In some embodiments, compared to the unmutated SB transposase as set forth in SEQ ID NO: 2, the amino acid sequence of the transposase polypeptide comprises an amino acid mutation selected from the group consisting of: G114A, S116A, K177A, and P178A.

For example, compared to the unmutated SB transposase amino acid sequence as set forth in SEQ ID NO: 2, the transposase polypeptide amino acid sequence comprises a mutation at amino acid position G114 to alanine (G114A). For example, compared to the unmutated SB transposase amino acid sequence as set forth in SEQ ID NO: 2, the transposase polypeptide amino acid sequence comprises a mutation at amino acid position S116 to alanine (S116A). For example, compared to the unmutated SB transposase amino acid sequence as set forth in SEQ ID NO: 2, the transposase polypeptide amino acid sequence comprises a mutation at amino acid position K177 to alanine (K177A). For example, compared to the unmutated SB transposase amino acid sequence as set forth in SEQ ID NO: 2, the transposase polypeptide amino acid sequence comprises a mutation at amino acid position P178 to alanine (P178A).

In some embodiments, compared to the unmutated SB transposase as set forth in SEQ ID NO: 2, the transposase polypeptide amino acid sequence comprises a combination of amino acid mutations selected from the group consisting of:
i) G114A, S116A;
ii) G114A, K177A;
iii) G114A, P178A;
iv) S116A, K177A;
v) S116A, P178A; and
vi) K177A, P178A.

In another aspect, the present application provides a transposase polypeptide comprising an amino acid sequence having at least about 90%, 91%, 92%, 93%, 94%, 95%, 96%, 97%, 98%, or 99% identity to SEQ ID NO: 2, wherein the transposase polypeptide comprises an alanine at the position corresponding to position 114 and/or an alanine at the position corresponding to position 116.

In some embodiments, the transposase polypeptide comprises an amino acid sequence as set forth in any one of SEQ ID NO: 1 or SEQ ID NO: 3-11, or a sequence having at least about 90%, 91%, 92%, 93%, 94%, 95%, 96%, 97%, 98%, or 99% identity to the amino acid sequence as set forth in any one of SEQ ID NO: 1 or SEQ ID NO: 3-11.

In another aspect, the present application provides a polynucleotide molecule comprising a nucleic acid sequence encoding the aforementioned transposase polypeptide.

In some embodiments, it includes RNA or DNA. In some aspects, the molecule can be a DNA expression vector. For example, the DNA expression vector may comprise a transposase coding sequence operably linked to a promoter (e.g., T7 or SP6 promoter) for in vitro polypeptide expression or to a promoter for polypeptide expression in mammalian cells. In some aspects, the polynucleotide molecule can be RNA or mRNA. In other aspects, the RNA may comprise a 5'-cap, an IRES motif, (heterologous) 5'UTR, (heterologous) 3'UTR, and/or a poly(A) sequence. In some aspects, the RNA may additionally comprise a poly(A) sequence of 20 to 300 nucleotides. In another aspect, the present application provides a method for preparing the aforementioned transposase polypeptide, comprising transfecting a cell with a polynucleotide encoding the transposase polypeptide and expressing the polypeptide from the polynucleotide. In another embodiment, the invention provides a host cell comprising the polypeptide or polynucleotide molecule of the embodiments. In some cases, the cell is a mammalian cell, such as a human cell. In some aspects, the cell is a stem cell or an induced pluripotent stem (iPS) cell. In further aspects, the cell is a natural killer (NK) cell, a precursor of NK cells, a T cell, a precursor of T cells, or an immune cell. In some cases, the cell may comprise RNA encoding the transposase polypeptide of the embodiments. In another embodiment, a population of cells is provided, wherein the cells comprise the polypeptide or polynucleotide molecule of the embodiments.

In another aspect, the present application provides a method for genetically engineering cells, comprising: transfecting cells with the aforementioned transposase polypeptide or a nucleic acid encoding the transposase polypeptide, along with a DNA vector comprising a sequence encoding a selected genetic element flanked by transposon repeat sequences, and then incubating the cells under conditions suitable for (transient or stable) transposase activity, thereby integrating the selected genetic element into the genome of the cells and producing engineered cells. In some aspects, the DNA vector encoding the selected genetic element flanked by transposon repeats further comprises a sequence encoding the transposase polypeptide of the embodiments. Thus, in some aspects, the method of the embodiments comprises transfecting cells with a DNA vector comprising a sequence encoding a selected genetic element flanked by transposon repeats and a sequence encoding the transposase of the embodiments (under the control of a promoter sequence), followed by incubating the cells under conditions suitable for transposase expression and activity, thereby integrating the selected genetic element into the genome of the cells and producing engineered cells. In some aspects, the method comprises a third step of isolating or culturing the engineered cells. In some aspects, the selected genetic element is a screenable or selectable marker. In other aspects, the selected genetic element may encode an antibody, an inhibitory nucleic acid (e.g., small interfering RNA (siRNA)), a therapeutic polypeptide, a T cell receptor (TCR), a chimeric antigen receptor (CAR), or an enhancer of immune cell function. In specific aspects, the selected genetic element encodes a CAR or a TCR. In other aspects, the selected genetic element may be a gene or a portion thereof used to replace or modify the corresponding gene from the cell (e.g., altering the sequence or expression of the gene or "knocking out" expression of the gene in the cell). In some aspects, the transfected cells are mammalian cells, such as human cells. In some instances, the cells may be stem cells or iPS cells. In some aspects, the cells may be immune system cells or their precursors, such as NK cells, T cells, precursors of NK cells, or precursors of T cells. In some aspects, transfecting cells may include using chemical-based transfection reagents, electroporation of cells, or other techniques providing delivery of nucleic acids and/or proteins into the cytoplasm and nucleus of the cells. For example, cells may be transfected using salt precipitates (e.g., CaPO4 precipitates), lipids (e.g., charged or nonpolar lipids), cationic polymers, PEG-complexes, and/or protein complexes (e.g., cationic polypeptides). In some aspects, transfection may involve the use of liposomes, such as phospholipid liposomes (e.g., liposomes incorporating glycerophospholipids or sphingolipids). In other aspects, cells may be transduced with viral vectors (e.g., adenovirus, adeno-associated virus, retrovirus (e.g., lentivirus), or vaccinia virus vectors). In some aspects, the viral vector used according to the embodiments is a non-integrating viral vector. Those skilled in the art will recognize that, in some aspects, the transposase of the embodiments may be delivered to cells together with or separately from nucleic acids encoding transposon repeats. For example, in certain aspects, the transposase may be delivered to cells as a recombinant polypeptide using protein transfection reagents, and the nucleic acid molecule containing transposon repeats may be delivered using a nucleic acid transfection system or a viral vector. In other aspects, RNA encoding the transposase is co-transfected with DNA containing transposon repeats and the selected genetic element.

In other aspects, the method further comprises transfecting a population of cells with the transposase polypeptide of the embodiments or nucleic acid encoding the transposase polypeptide, and a DNA vector comprising a sequence encoding the selected genetic element flanked by transposon repeats, and incubating the population under conditions suitable for transposase activity, thereby integrating the selected genetic element into the genome of the cells and generating a population of engineered cells. In specific aspects, the method comprises transfecting a population of T cells or T cell precursors with the transposase polypeptide or nucleic acid encoding the transposase polypeptide, and a DNA vector comprising a sequence encoding a CAR flanked by transposon repeats, and incubating the population under conditions suitable for transposase activity, thereby integrating the CAR into the genome of the cells and generating a population of engineered T cells or T cell precursors. In other aspects, the method comprises transfecting a population of T cells or T cell precursors with a DNA vector comprising a sequence encoding a CAR flanked by transposon repeats and a sequence encoding the transposase of the embodiments (operably linked to a promoter), and incubating the population under conditions suitable for transposase activity, thereby integrating the CAR into the genome of the cells and generating a population of engineered T cells or T cell precursors. In some aspects, the engineered cells may additionally be cultured in a medium that selectively enhances proliferation of CAR-expressing T cells.

In another embodiment, a method is provided for eliciting a T cell response in a human subject having a disease, which comprises first obtaining a population of engineered T cells or T cell precursors according to the embodiments, optionally culturing the cells in a medium that selectively enhances proliferation of CAR-expressing T cells, and then administering an effective amount of CAR-expressing T cells to the subject to elicit a T cell response.

In some aspects, the methods of the embodiments comprise: (a) obtaining a cell sample from a subject, wherein the sample comprises T cells or T cell progenitors; (b) transfecting the cells with DNA encoding a chimeric antigen receptor (CAR) flanked by transposons and a transposase of the embodiments effective to integrate the DNA encoding the CAR into the genome of the cells to provide a population of transgenic CAR-expressing cells; (c) optionally culturing the transgenic CAR cell population ex vivo in a medium that selectively enhances proliferation of CAR-expressing T cells; and (d) administering an effective amount of the transgenic CAR cells to the subject to elicit a T cell response. Thus, in some aspects, the transgenic CAR cells are cultured ex vivo for less than 21 days, for example, less than 20, 19, 18, 17, 16, 15, 14, 13, 12, 11, 10, 9, 8, 7, 6, 5, 4, 3, 2 days, or fewer. In certain aspects, the CAR cells are cultured ex vivo for no more than 3 to 5 days. In other aspects, steps (a)-(d) of the method (i.e., obtaining the cell sample to administration of CAR T cells) are completed within no more than 21, 20, 19, 18, 17, 16, 15, 14, 13, 12, 11, 10, 9, 8, 7, 6, or 5 days. In other aspects, the cell sample from the subject may be a sample of peripheral blood or umbilical cord blood of less than about 200 ml. In some aspects, the sample may be collected by apheresis. In certain aspects, the sample is collected by a method not involving apheresis (e.g., by venipuncture). In other aspects, the initial volume of the cell sample is less than 175 ml, less than about 175 ml, less than 150 ml, less than about 150 ml, less than 125 ml, less than about 125 ml, less than 100 ml, less than about 100 ml, less than 75 ml, less than about 75 ml, less than 50 ml, less than about 50 ml, less than 25 ml, or less than about 25 ml (e.g., when obtained from the subject, the cell sample has an initial volume between about 50 and about 200 ml, between about 50 and about 100 ml, or between about 100 and about 200 ml).

In some aspects, the methods of the embodiments involve transfecting cells with DNA encoding a chimeric antigen receptor (CAR) and a transposase. Cell transfection methods are well known in the art, but in certain aspects, highly efficient transfection methods such as electroporation are employed. For example, a nucleofection device may be used to introduce nucleic acids into cells. In certain embodiments, the transfection step does not involve infection or transduction of cells with a virus, which could cause genotoxicity and/or elicit an immune response in the treated subject due to the presence of viral sequences in the cells.

Other aspects of the embodiments involve transfecting cells with an expression vector encoding a CAR. A large number of CAR constructs and their expression vectors are known in the art. For example, in some aspects, the CAR expression vector is a DNA expression vector, such as a plasmid, linear expression vector, or episome. In some aspects, the vector contains additional sequences, such as sequences promoting CAR expression, including promoters, enhancers, poly-A signals, and/or one or more introns. In some aspects, the CAR-encoding sequence is flanked by transposon sequences such that the presence of transposase allows integration of the encoding sequence into the genome of the transfected cells.

As detailed above, in certain aspects, cells are further transfected with the transposase of the embodiments that promotes integration of the CAR-encoding sequence into the genome of the transfected cells. In some aspects, the transposase is provided as a DNA expression vector. In certain aspects, the transposase is provided as expressible RNA or protein, such that long-term expression of the transposase does not occur in the transgenic cells. For example, in some aspects, transposase encoded by mRNA (e.g., mRNA containing a cap and poly-A tail) is provided.

The methods of the embodiments can be used to prepare (e.g., for clinical trials) CAR+ T cells having binding specificity for various tumor antigens (e.g., CD19, ROR1, CD56, EGFR, CD33, CD123, c-met, GD2). CAR+ T cells produced using this technology can be used to treat patients suffering from leukemia (e.g., AML, ALL, CML), infections, and/or solid tumors. For example, the methods of the embodiments can be used to treat cell proliferative diseases, fungal, viral, bacterial, or parasitic infections. Pathogens that can be targeted include, but are not limited to, Plasmodium, Trypanosoma, Aspergillus, Candida, HSV, RSV, EBV, CMV, JC virus, BK virus, or Ebola pathogen. Other examples of antigens targetable by CAR cells of the embodiments include, but are not limited to, CD19, CD20, carcinoembryonic antigen, alpha-fetoprotein, CA-125, 5T4, MUC-1, epithelial tumor antigen, melanoma-associated antigen, mutant p53, mutant ras, HER2/Neu, ERBB2, folate-binding protein, HIV-1 envelope glycoprotein gp120, HIV-1 envelope glycoprotein gp41, GD2, CD123, CD23, CD30, CD56, c-Met, mesothelin, GD3, HERV-K, IL-11Rα, kappa chain, lambda chain, CSPG4, ERBB2, EGFRvIII, or VEGFR2.

In some embodiments of the present invention, CAR cells are delivered to an individual in need thereof, such as an individual having cancer or an infection. The cells then enhance the individual's immune system to attack the corresponding cancerous or pathogenic cells. In some cases, the individual is administered one or more doses of antigen-specific CAR T cells. In cases where the individual receives two or more doses of antigen-specific CAR T cells, the interval between administrations should be sufficient to allow time for proliferation in the individual, and in specific embodiments, the interval between doses is 1, 2, 3, 4, 5, 6, 7 days, or more.

The source of allogeneic or autologous T cells modified to contain a chimeric antigen receptor (and, in some cases, lacking a functional TCR) may be of any type, but in specific embodiments, for example, the cells are obtained from umbilical cord blood, peripheral blood, human embryonic stem cells, or induced pluripotent stem cell banks. A suitable dose for therapeutic effect would be at least 105 cells per dose or between about 105 and about 1010 cells, for example, preferably administered in a series of dosing cycles. An exemplary dosing regimen consists of four weekly dosing cycles with escalating doses, starting on day 0 with at least about 105 cells, for example, gradually increasing over several weeks of an intra-patient dose escalation scheme to a target dose of about 1010 cells. Suitable routes of administration include intravenous, subcutaneous, intracavitary (e.g., via a reservoir device), intraperitoneal, and direct injection into a tumor mass. The pharmaceutical compositions of the present invention may be used alone or in combination with other established agents useful for treating cancer. Whether delivered alone or in combination with other agents, the pharmaceutical compositions of the present invention may be delivered via various routes to various sites within mammals, particularly humans, to achieve specific effects. Those skilled in the art will recognize that although more than one route may be used for administration, a particular route may provide a more direct and more effective response than another route. For example, intradermal delivery may be more advantageous than inhalation for treating melanoma. Local or systemic delivery may be achieved by administration, including application or instillation of the formulation into a body cavity, inhalation or insufflation of an aerosol, or parenteral introduction, including intramuscular, intravenous, intraportal, intrahepatic, intraperitoneal, subcutaneous, or intradermal administration.

The compositions of the embodiments may be provided in unit dosage form, wherein each dosage unit (e.g., an injection) contains a predetermined amount of the composition alone or appropriately combined with other active agents. As used herein, the term "unit dosage form" refers to a physically discrete unit suitable as a unit dose for human and animal subjects, each unit containing a predetermined amount of the composition of the present invention alone or in combination with other active agents, calculated in an amount sufficient to produce the desired effect when combined with a pharmaceutically acceptable diluent, carrier, or vehicle, as appropriate. The specifications of the unit dosage forms of the present invention depend on the specific pharmacodynamics associated with the pharmaceutical composition in the particular subject.

An effective amount or sufficient number of isolated transduced T cells is present in the composition and introduced into the subject such that a long-term, specific anti-tumor response is established to reduce tumor size or eliminate tumor growth or regrowth compared to what would otherwise occur in the absence of such treatment. Ideally, compared to the original or initial (e.g., "day 0 of treatment") tumor size, the amount of transduced T cells reintroduced into the subject results in a tumor size reduction of approximately or at least about 10%, approximately or at least about 20%, approximately or at least about 30%, approximately or at least about 40%, approximately or at least about 50%, approximately or at least about 60%, approximately or at least about 70%, approximately or at least about 80%, approximately or at least about 90%, approximately or at least about 95%, approximately or at least about 98%, or approximately 100% or 100%.

Any of the compositions described herein may be included in a kit. In some aspects, the transposase polypeptide of the embodiments or nucleic acid encoding the transposase polypeptide is provided in the kit. Such kits may include various additional components, such as DNA vectors encoding transposon repeats, transfection reagents, cells, CAR expression constructs, media, aAPCs, growth factors, antibodies (e.g., for sorting or characterizing CAR T cells), and/or plasmids encoding CAR or transposase.

In a non-limiting example, the kit contains the transposase polypeptide of the embodiments or nucleic acid encoding the transposase polypeptide, one or more reagents for producing a CAR-expressing construct (with flanking transposon repeats), cells for transfecting the expression construct, and/or one or more instruments for obtaining cells for transfecting the expression construct (such instruments may be syringes, pipettes, forceps, and/or any such medically approved devices). In other aspects, transfection devices such as electroporation devices are included.

The kit may contain one or more appropriately aliquoted compositions of the present invention or reagents for producing the compositions of the present invention. The components of the kit may be packaged in aqueous medium or in lyophilized form. The container means of the kit may include at least one vial, tube, flask, bottle, syringe, or other container means into which the composition may be placed and preferably appropriately aliquoted. In cases where the kit contains multiple components, the kit will generally also include a second, third, or other additional container for separately placing the additional components. However, various combinations of components may be included in vials. Kits of the present invention will generally also include means for containing the chimeric receptor construct and any other reagent containers in a commercially saleable sealed enclosure. For example, such containers may include injection-molded or blow-molded plastic containers retaining the desired vials.

Without being bound by any theory, the examples below are provided solely to illustrate the transposase polypeptides, preparation methods, and uses of the present application and are not intended to limit the scope of the invention of the present application.

### Examples

### Example 1 Transposase Cleavage Activity Assay

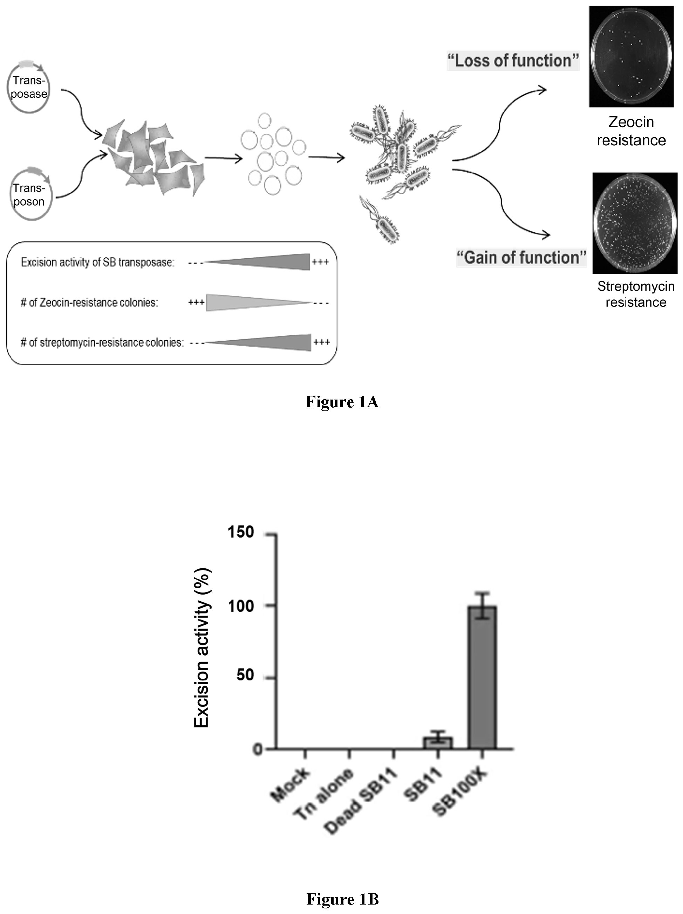

Figure 1A shows a schematic diagram of the transposase cleavage activity assay. The streptomycin resistance gene in the plasmid is interrupted by a transposon ecoding a Zeocin resistance gene. HEK293T cells were co-transfected with this plasmid and a transposase plasmid. Plasmid DNA was extracted from HEK293T cells and used to transform E. coli. Plasmids cleaved by the transposase will lose Zeocin resistance but gain streptomycin resistance.
Figure 1B shows the results of the assay for cleavage activity of different transposases. HEK293T cells were seeded in 6-well plates and cultured overnight, then transfected with equal amounts of transposon plasmid plus either no transposase, an inactivated transposase mutant (E279D), SB11 transposase, or SB100X transposase plasmid. After 72 hours, cells were collected, plasmid DNA was extracted using a Plasmid Mini Kit, and E. coli was transformed. Transformed bacteria were plated on streptomycin-containing plates, cultured overnight, and single colony-forming units were counted. The number of colonies reflects transposase activity. The cleavage activity of SB100X transposase is higher than that of SB11, whereas the transposon alone or the inactivated transposase mutant showed no cleavage activity. Plasmid DNA extracted from the colonies was sequenced, further confirming that the streptomycin-disrupted reading frame on the transposon was restored upon transposase action.

### Example 2 Transposase Mutants Generated by Alanine Scanning Exhibit Different Cleavage Activities

Transposase mutants were generated by site-directed mutagenesis PCR. After sequencing verification, plasmids encoding different transposase mutants were co-transfected with a transposon plasmid into HEK293T cells, and transposase cleavage activity was assayed using the method described in Example 1. The E279D transposase mutant served as a negative control. Results were normalized to SB32X transposase (SEQ ID NO: 2). All error bars represent S.E.M. of three independent replicates. The results show that multiple transposase mutants provided in the present application exhibit higher cleavage activity than SB32X.

### Example 3 Transposition Activity Assay of Transposase Mutants

HeLa cells were cultured in DMEM (Gibco, NY, USA) supplemented with fetal bovine serum. Cells were co-transfected with 250 ng of plasmid DNA encoding each transposase mutant and 750 ng of transposon plasmid DNA encoding a Zeocin resistance gene. After 24 hours, the cells were transferred to a 10-cm culture dish. Resistant cell clones were simultaneously selected using medium containing Zeocin. Two weeks later, the cells were fixed with 4% paraformaldehyde, stained, and the number of cell clones was counted. Results were normalized to SB32X transposase (SEQ ID NO: 2). All error bars represent the S.E.M. of three independent replicates. The results show that multiple transposase mutants provided in this application exhibit higher nuclease activity than SB32X.

### Example 4 Preparation of CAR-T cells using transposase mutants

T cells were isolated from PBMCs using magnetic beads, and minicircle DNA expressing CD19-CAR was co-electroporated into T cells together with plasmids encoding one of three transposases-SB11, SB100X, or Tri-FITase-using an electroporator (Lonza). After overnight culture in RPMI-1640 medium supplemented with 10% FBS and 0.1% DMSO, viable cells were enriched by density gradient centrifugation using OptiPrep (Axis-Shield). CAR-T cells were then expanded in X-VIVO15 medium containing 10% FBS and cytokines (50 IU/mL IL-2, 50 IU/mL IL-7, and 100 IU/mL IL-15), with the medium refreshed every two days. Viable cell counts were determined at various time points after electroporation, and CAR-T positivity rate and MFI were assessed by flow cytometry. Dead cells were excluded using 7-AAD; T cells were labeled with PE-Cy7-Anti-CD3, and CAR-T cells were labeled with FITC-CD19-Fc. T cells transfected with different transposases maintained high viability. The percentage of CAR-positive T cells transfected with Tri-FITase was higher than that of T cells transfected with SB11 transposase, and similar to that of T cells transfected with SB100X.

## Claims

1. A transposase polypeptide, wherein the amino acid sequence of said transposase polypeptide comprises at least one mutated amino acid residue, as compared to the amino acid sequence from position 100 to position 180 of the unmutated SB transposase as set forth in SEQ ID NO: 2.

2. The transposase polypeptide according to claim 1, wherein the amino acid sequence of said transposase polypeptide comprises 1 to 20 mutated amino acid residues.

3. The transposase polypeptide according to claim 1, wherein the amino acid sequence of said transposase polypeptide comprises at least two mutated amino acid residues.

4. The transposase polypeptide according to any one of claims 1-3, wherein the mutation site of said at least one mutated amino acid residue is located within positions 110 to 120 and/or positions 170 to 180 of the amino acid sequence as set forth in SEQ ID NO: 2.

5. The transposase polypeptide according to any one of claims 1-4, wherein said mutated amino acid residue is located at position 114, position 116, position 177, and/or position 178 of the amino acid sequence as set forth in SEQ ID NO: 2.

6. The transposase polypeptide according to any one of claims 1-5, wherein the amino acid sequence of said transposase polypeptide comprises a mutation at amino acid position G114, as compared to the amino acid sequence of the unmutated SB transposase as set forth in SEQ ID NO: 2.

7. The transposase polypeptide according to any one of claims 1-6, wherein the amino acid sequence of said transposase polypeptide comprises a mutation at amino acid position S116, as compared to the amino acid sequence of the unmutated SB transposase as set forth in SEQ ID NO: 2.

8. The transposase polypeptide according to any one of claims 1-7, wherein the amino acid sequence of said transposase polypeptide comprises a mutation at amino acid position K177, as compared to the amino acid sequence of the unmutated SB transposase as set forth in SEQ ID NO: 2.

9. The transposase polypeptide according to any one of claims 1-8, wherein the amino acid sequence of said transposase polypeptide comprises a mutation at amino acid position P178, as compared to the amino acid sequence of the unmutated SB transposase as set forth in SEQ ID NO: 2.

10. The transposase polypeptide according to any one of claims 1-9, wherein said at least one mutated amino acid residue is mutated to an alanine residue.

11. The transposase polypeptide according to any one of claims 1-10, wherein all of said amino acid residue mutations are mutated to alanine residues.

12. The transposase polypeptide according to any one of claims 1-11, wherein the amino acid sequence of said transposase polypeptide comprises the amino acid mutation selected from the group consisting of: G114A, S116A, K177A, and P178A, as compared to the amino acid sequence of the unmutated SB transposase as set forth in SEQ ID NO: 2.

13. The transposase polypeptide according to any one of claims 1-12, wherein the amino acid sequence of said transposase polypeptide comprises a mutation at amino acid position G114 to alanine (G114A), as compared to the amino acid sequence of the unmutated SB transposase as set forth in SEQ ID NO: 2.

14. The transposase polypeptide according to any one of claims 1-13, wherein the amino acid sequence of said transposase polypeptide comprises a mutation at amino acid position S116 to alanine (S116A), as compared to the amino acid sequence of the unmutated SB transposase as set forth in SEQ ID NO: 2.

15. The transposase polypeptide according to any one of claims 1-14, wherein the amino acid sequence of said transposase polypeptide comprises a mutation at amino acid position K177 to alanine (K177A), as compared to the amino acid sequence of the unmutated SB transposase as set forth in SEQ ID NO: 2.

16. The transposase polypeptide according to any one of claims 1-15, wherein the amino acid sequence of said transposase polypeptide comprises a mutation at amino acid position P178 to alanine (P178A), as compared to the amino acid sequence of the unmutated SB transposase as set forth in SEQ ID NO: 2.

17. The transposase polypeptide according to any one of claims 1-16, wherein the amino acid sequence of said transposase polypeptide comprises a combination of amino acid mutations selected from the group consisting of:
i) G114A, S116A;
ii) G114A, K177A;
iii) G114A, P178A;
iv) S116A, K177A;
v) S116A, P178A; and
vi) K177A, P178A.

18. A transposase polypeptide, comprising a sequence having at least 90% identity to SEQ ID NO: 2, wherein said transposase polypeptide comprises an alanine at the position corresponding to position 114 and/or an alanine at the position corresponding to position 116.

19. The transposase polypeptide according to any one of claims 1-18, wherein said transposase polypeptide comprises the amino acid sequence as set forth in any one of SEQ ID NO: 1 or SEQ ID NO: 3-11.

20. A polynucleotide molecule, comprising a nucleic acid sequence encoding the transposase polypeptide according to any one of claims 1-19.

21. The polynucleotide molecule according to claim 20, comprising RNA or DNA.

22. The polynucleotide molecule according to any one of claims 20-21, wherein said DNA comprises a promoter for expression of said transposase polypeptide.

23. The polynucleotide molecule according to any one of claims 20-22, wherein said DNA comprises a promoter for expression of said transposase polypeptide in mammalian cells.

24. The polynucleotide molecule according to any one of claims 20-21, wherein said molecule is mRNA.

25. The polynucleotide molecule according to claim 24, wherein said polynucleotide molecule comprises a 5' cap, an IRES motif, and/or a poly(A) sequence.

26. The polynucleotide molecule according to any one of claims 20-25, wherein said polynucleotide molecule comprises a poly(A) sequence of 20 to 300 nucleotides.

27. A construct, comprising the polynucleotide molecule according to any one of claims 20-26 and a regulatory sequence, wherein said regulatory sequence is operably linked to said expressible polynucleotide to allow expression of said polynucleotide.

28. A cell, comprising the transposase polypeptide according to any one of claims 1-19, the polynucleotide molecule according to any one of claims 20-26, and/or the construct according to claim 27.

29. The cell according to claim 28, wherein said cell comprises a mammalian cell.

30. The cell according to any one of claims 28-29, wherein said cell comprises a human cell.

31. The cell according to any one of claims 28-30, wherein said cell is autologous.

32. The cell according to any one of claims 28-31, wherein said cell is allogeneic.

33. The cell according to any one of claims 28-32, wherein said cell comprises a stem cell or an induced pluripotent stem (iPS) cell.

34. The cell according to any one of claims 28-33, wherein said cell comprises an immune effector cell.

35. The cell according to any one of claims 28-34, wherein said cell comprises a natural killer (NK) cell, a T cell, or a precursor of an NK cell or a T cell.

36. The cell according to any one of claims 28-35, wherein said cell comprises the transposase polypeptide according to any one of claims 1-19.

37. The cell according to any one of claims 28-36, wherein said cell comprises mRNA or DNA encoding the transposase polypeptide according to any one of claims 1-19.

38. A transposon system, comprising:
(a) a transposon unit comprising an inverted terminal repeat (ITR) or a direct terminal repeat (DTR), wherein said inverted terminal repeat (ITR) or direct terminal repeat (DTR) flanks a target sequence to be inserted into the genome of a target cell; and
(b) the transposase polypeptide according to any one of claims 1-19, the polynucleotide molecule according to any one of claims 20-26, and/or the construct according to claim 27.

39. Use of the transposon system according to claim 38 for gene delivery to target cells in vitro.

40. A method of genetically engineering a cell, comprising the steps of:
(a) transfecting a target cell with the transposon system according to claim 38;
(b) culturing the target cell under conditions permitting culture of the target cell to produce an engineered cell.

41. The method according to claim 40, wherein said method further comprising: (c) isolating said engineered cells.

42. The method according to any one of claims 40-41, wherein said target sequence encodes a screenable or selectable marker.

43. The method according to any one of claims 40-42, wherein said target sequence encodes a therapeutic polypeptide or an inhibitory nucleic acid.

44. The method according to any one of claims 40-43, wherein said target sequence encodes an antibody, a T cell receptor (TCR), a chimeric antigen receptor (CAR).

45. The method according to any one of claims 40-44, wherein said target sequence encodes a CAR.

46. The method according to claim 45, wherein said CAR targets a tumor antigen.

47. The method according to claim 46, wherein said tumor antigen is CD19, CD20, ROR1, CD22 carcinoembryonic antigen, alpha-fetoprotein, CA-125, 5T4, MUC-1, epithelial tumor antigen, prostate-specific antigen, melanoma-associated antigen, mutated p53, mutated ras, HER2/Neu, folate-binding protein, HIV-1 envelope glycoprotein gp120, HIV-1 envelope glycoprotein gp41, GD2, CD123, CD33, CD138, CD23, CD30, CD56, c-Met, mesothelin, GD3, HERV-K, IL-11Rα, κ chain, λ chain, CSPG4, ERBB2, EGFRvIII, VEGFR2, a combination of HER2-HER3, or a combination of HER1-HER2.

48. The method according to any one of claims 40-47, wherein said target cell comprises a mammalian cell.

49. The method according to any one of claims 40-48, wherein said target cell comprises a human cell.

50. The method according to any one of claims 40-49, wherein said target cell is autologous.

51. The method according to any one of claims 40-50, wherein said target cell is allogeneic.

52. The method according to any one of claims 40-51, wherein said target cell comprises a stem cell or an induced pluripotent stem (iPS) cell.

53. The method according to any one of claims 40-52, wherein said target cell comprises an immune effector cell or a precursor thereof.

54. The method according to any one of claims 40-53, wherein said target cell comprises a natural killer (NK) cell, a T cell, or a precursor of an NK cell or a T cell.

55. The method according to any one of claims 40-54, wherein transfecting said target cell comprises using a polymer-based, polypeptide-based or lipid-based transfection reagent.

56. The method according to any one of claims 40-55, wherein transfecting said target cell comprises electroporating said cell.

57. The method according to any one of claims 40-56, wherein said method further comprises:
(a) transfecting a population of cells with the transposon system according to claim 38;
(b) incubating said population under conditions suitable for transposase activity, thereby integrating said target sequence into the genome of said cells and producing a population of engineered cells.

58. The method according to any one of claims 40-57, wherein said method further comprises:
(a) transfecting a population of T cells or T cell precursors with the transposon system according to claim 38, wherein said target sequence encodes a CAR or a TCR;
(b) incubating said population under conditions suitable for transposase activity, thereby integrating said CAR into the genome of said cells and producing a population of engineered T cells or T cell precursors.

59. The method according to claim 58, wherein said method further comprises: (c) culturing said engineered cells in a medium that selectively enhances proliferation of CAR- or TCR-expressing T cells.

60. A pharmaceutical composition, comprising the transposase polypeptide according to any one of claims 1-19, the polynucleotide molecule according to any one of claims 20-26, and/or the construct according to claim 27, and a pharmaceutically acceptable carrier and/or excipient.

61. A kit, comprising
(a) a transposon unit containing inverted terminal repeats (ITRs) or DTRs, wherein said inverted terminal repeats (ITRs) or DTRs flank a target sequence to be inserted into the genome of a target cell; and
(b) the transposase polypeptide according to any one of claims 1-19, the polynucleotide molecule according to any one of claims 20-26, and/or the construct according to claim 27.

62. Use of the transposase polypeptide according to any one of claims 1-19, the polynucleotide molecule according to any one of claims 20-26, the construct according to claim 27, the cell according to any one of claims 28-37, the transposon system according to claim 38, and/or the pharmaceutical composition according to claim 60 in the manufacture of a medicament for treating a disease or disorder.

63. The use according to claim 62, wherein said disease or disorder is cancer.

64. The use according to claim 62, wherein said disease or disorder is an infectious disease.

65. A compound for treating a disease or disorder, wherein said compound comprises the transposase polypeptide according to any one of claims 1-19, the polynucleotide molecule according to any one of claims 20-26, the construct according to claim 27, the cell according to any one of claims 28-37, the transposon system according to claim 38, the pharmaceutical composition according to claim 60, and/or the kit according to claim 61.
